# EUROPÄISCHE PATENTSCHRIFT

(11) **EP 2 685 907 B1**
(45) Veröffentlichungstag und Bekanntmachung des Hinweises auf die Patenterteilung: **31.10.2018**
(21) Anmeldenummer: 12707767.5
(22) Anmeldetag: 08.03.2012
(51) Int. Cl.: A61B 17/072, A61B 18/14

(54) **CHIRURGISCHES SYSTEM ZUM VERBINDEN VON KÖRPERGEWEBE**
SURGICAL SYSTEM FOR CONNECTING BODY TISSUE
SYSTÈME CHIRURGICAL DESTINÉ À RELIER DES TISSUS CORPORELS

(30) Priorität: 17.03.2011 DE 102011001372
(43) Veröffentlichungstag der Anmeldung: 22.01.2014
(73) Patentinhaber: Aesculap AG, 78532 Tuttlingen (DE)
(72) Erfinder: WEISSHAUPT, Dieter, 78194 Immendingen (DE); ROTHWEILER, Christoph, 78166 Donaueschingen (DE); HEIZMANN, Patrick, 78183 Hüfingen (DE)
(74) Vertreter: Winter, Brandl, Fürniss, Hübner, Röss, Kaiser, Polte - Partnerschaft mbB
(86) Internationale Anmeldenummer: PCT/EP2012/054047
(87) Internationale Veröffentlichungsnummer: WO 2012/123337

(56) Entgegenhaltungen:
- EP-A2- 0 741 996
- WO-A1-03/094745
- WO-A2-2010/088044
- US-A1- 2005 107 784
- US-A1- 2007 173 811
- US-A1- 2010 213 238

## Beschreibung

Die vorliegende Erfindung betrifft ein chirurgisches System zum Verbinden von Körpergewebe, umfassend ein chirurgisches Instrument mit mindestens zwei relativ zueinander bewegbaren Werkzeugelementen, welche jeweils mindestens eine HF-Elektrode umfassen, die in einer Annäherungsstellung der Werkzeugelemente einen minimalen Abstand voneinander definieren, einander gegenüberliegen und aufeinander zu weisen, um infolge einer Beaufschlagung mit einem HF-Strom zwischen den Werkzeugelementen gehaltenes Körpergewebe zu verbinden, und mit einem Schneidelement zum Durchtrennen und/oder Abtrennen von Körpergewebe, welches Schneidelement von einer Gewebeverbindungsstellung, in welcher Körpergewebe mit den Werkzeugelementen verbindbar ist und in welcher das Schneidelement nicht mit Körpergewebe in Kontakt treten kann, in eine Schneidstellung bringbar ist.

Ferner betrifft die vorliegende Offenbarung ein Steuerungsverfahren für ein chirurgisches System zum Verbinden von Körpergewebe, welches System ein chirurgisches Instrument umfasst mit mindestens zwei relativ zueinander bewegbaren Werkzeugelementen, welche jeweils mindestens eine HF-Elektrode umfassen, die in einer Annäherungsstellung der Werkzeugelemente einen minimalen Abstand voneinander definieren, einander gegenüberliegen und aufeinander zu weisen, um infolge einer Beaufschlagung mit einem HF-Strom zwischen den Werkzeugelementen gehaltenes Körpergewebe zu verbinden, und mit einem Schneidelement zum Durchtrennen und/oder Abtrennen von Körpergewebe, welches Schneidelement von einer Gewebeverbindungsstellung, in welcher Körpergewebe mit den Werkzeugelementen verbindbar ist und in welcher das Schneidelement nicht mit Körpergewebe in Kontakt treten kann, in eine Schneidstellung bringbar ist.

Chirurgische Systeme und Steuerungsverfahren der eingangs beschriebenen Art sind beispielsweise aus der DE 20 2010 013 150 U1 bekannt. Sie kommen insbesondere bei chirurgischen Eingriffen zum Einsatz, bei denen sogenannte "Side-to-Side"-, "End-to-End"- oder "End-to-Side"-Anastomosen durchgeführt werden. Zum Verbinden des Körpergewebes werden die miteinander zu verbindenden Teile mit einem HF-Strom definierter Stärke beaufschlagt. Die so hergestellte Verbindung wird auch als "Verschweißung" beziehungsweise "Versiegelung" bezeichnet.

Weiterer gattungsgemäßer Stand der Technik ist aus den Dokumenten EP 0 741 996 A2, US 2005 / 107784 A1 und US 2007/173811 A1 bekannt.

Nach der Verbindung des Gewebes kann mittels des Schneidelements überschüssiges Gewebe abgetrennt oder die nun verbundenen Gewebeteile zum Eröffnen insbesondere einer neu ausgebildeten Kavität wieder teilweise durchtrennt werden. Wichtig dabei ist, dass das Schneidelement erst dann aktiviert wird, wenn die Verbindung des Körpergewebes abgeschlossen ist. Andernfalls kann es zu einer unerwünschten Schädigung des Patienten im Bereich des Operationssitus kommen.

Es ist daher eine Aufgabe der vorliegenden Erfindung, ein chirurgisches System der eingangs beschriebenen Art so zu verbessern, dass eine Fehlbedienung des Systems möglichst ausgeschlossen ist.

Diese Aufgabe wird bei einem chirurgischen System der eingangs beschriebenen Art erfindungsgemäß dadurch gelöst, dass das Instrument eine Sicherungseinrichtung gemäß Anspruch 1 umfasst zum Sichern des Schneidelements in der Gewebeverbindungsstellung.

Die Sicherungseinrichtung ermöglicht es insbesondere, den Einsatz des Schneidelements zu verhindern, solange ein Durchtrennen oder Abtrennen von Körpergewebe nicht oder noch nicht erwünscht ist. Dies ermöglicht es Operateuren, die "Side-to-Side"-, "End-to-End"- beziehungsweise "End-to-Side"-Anastomosen bislang mit herkömmlichen Klammernahtgeräten hergestellt haben, ein chirurgisches System zum Verbinden von Körpergewebe unter Einsatz eines HF-Stroms mit gleicher Handhabung anzubieten. Operateure, die Klammernahtgeräte verwenden, sind es gewohnt, zunächst das Gewebe mit dem Klammernahtgerät zu fassen, dann die zu verbindenden Gewebeteile mit Klammern zu verbinden und nach dem klammernden Verbinden des Körpergewebes dieses automatisch mit einem Schneidelement des Klammernahtgeräts mechanisch zu durchtrennen. Durch den konstruktiven Aufbau herkömmlicher Klammernahtgeräte ist sichergestellt, dass das Schneidelement erst dann aktiviert werden kann, wenn mindestens eine Klammer gesetzt ist. Die erfindungsgemäß vorgeschlagene Sicherungseinrichtung kann insbesondere sicherstellen, dass nun auch die Handhabung eines eingangs beschriebenen chirurgischen Systems der Handhabung eines Klammernahtgeräts entsprechen kann, was insbesondere deshalb wichtig ist, da sich die Geräte zum Verbinden von Körpergewebe mit HF-Strom äußerlich nur wenig von herkömmlichen Klammernahtgeräten unterscheiden und ein Operateur daher ein mit HF-Strom betriebenes chirurgisches Instrument intuitiv gleich benutzt wie ein Klammernahtgerät.

Besonders einfach wird der Aufbau des chirurgischen Systems, wenn die Sicherungseinrichtung mindestens ein Sicherungselement umfasst, welches in der Gewebeverbindungsstellung eine Aktivierung des Schneidelements blockiert oder verhindert. Das mindestens eine Sicherungselement kann insbesondere auf einfache Weise sicherstellen, dass das Schneidelement nicht in unerwünschter Weise bewegt wird oder mit einem HF-Strom zum Schneiden des Körpergewebes beaufschlagt wird, ohne dass dies tatsächlich gewollt ist.

Auf besonders einfache Weise lässt sich das Schneidelement in der Gewebeverbindungsstellung dadurch sichern, dass das mindestens eine Sicherungselement in der Gewebeverbindungsstellung mit dem Schneidelement mindestens teilweise kraft- und/oder formschlüssig in Eingriff steht. Mit anderen Worten sichert die Sicherungseinrichtung somit das Schneidelement insbesondere mechanisch in der Gewebeverbindungsstellung.

Eine besonders einfache Konstruktion des Systems kann insbesondere dadurch erreicht werden, dass die Sicherungseinrichtung ein erstes und ein zweites Sicherungselement umfasst, dass das Schneidelement das erste Sicherungselement umfasst oder trägt und dass das Instrument das zweite Sicherungselement umfasst oder trägt. Beide Sicherungselemente können somit am Instrument vorgesehen sein, wobei das erste Sicherungselement vorzugsweise am Schneidelement angeordnet oder ausgebildet ist.

Vorteilhaft kann es ferner sein, wenn das erste und das zweite Sicherungselement relativ zueinander bewegbar sind von einer Sicherungsstellung, in welcher sie miteinander kraft- und/oder formschlüssig in Eingriff stehen, in eine Freigabestellung, in welcher sie außer Eingriff stehen. Diese Ausgestaltung ermöglicht es, durch einfache Bewegung der Sicherungselemente relativ zueinander das Schneidelement entweder in der Gewebeverbindungsstellung zu sichern oder das Schneidelement freizugeben, um Körpergewebe abzutrennen oder zu durchtrennen.

Besonders vorteilhaft ist, wenn das erste und das zweite Sicherungselement relativ zueinander in einer Richtung bewegbar sind, welche quer, vorzugsweise senkrecht, zu einer Bewegungsrichtung des Schneidelements beim Übergang von der Gewebeverbindungsstellung in eine Gewebetrennstellung, in welcher das Schneidelement mindestens abschnittsweise über mindestens eines der mindestens zwei Werkzeugelemente vorsteht, verläuft, um Körpergewebe zu durchtrennen. Durch die quer zueinander orientierten Bewegungsrichtungen der Sicherungselemente relativ zueinander sowie des Schneidelements, kann auf einfache Weise eine besonders sichere Arretierung des Schneidelements in der Gewebeverbindungsstellung erreicht werden.

Die Ausgestaltung der Sicherungseinrichtung wird besonders einfach, wenn eines der beiden Sicherungselemente in Form eines Vorsprungs und wenn das andere Sicherungselement in Form einer Ausnehmung ausgebildet ist. Insbesondere können die beiden Sicherungselemente korrespondierend zueinander ausgebildet sein, so dass ein kraft- und/oder formschlüssiges Zusammenwirken der Sicherungselemente in der Gewebeverbindungsstellung ermöglicht wird. Grundsätzlich können beide Teile am Instrument beweglich angeordnet sein, beispielsweise der Vorsprung oder aber auch ein die Ausnehmung tragendes Teil des Instruments.

Auf besonders einfache Weise lässt sich die Sicherungseinrichtung betätigen, wenn eines der beiden Sicherungselemente mit einem am Instrument angeordneten Antrieb zum Bewegen des Sicherungselements gekoppelt ist. Mit dem Antrieb kann somit mindestens eines der Sicherungselemente bewegt werden, um diese relativ zueinander so zu bewegen, dass die Sicherungseinrichtung von der Sicherungsstellung in die Freigabestellung überführbar ist.

Günstig ist es, wenn der Antrieb ein elektromotorischer, ein pneumatischer, ein hydraulischer, ein magnetischer, ein elektro-magnetischer oder ein piezoelektrischer Antrieb ist. Mit derartigen Antrieben lässt sich ein Sicherungselement am Instrument auf einfache und sichere Weise definiert bewegen.

Vorzugsweise umfasst der Antrieb einen Magneten, einen Elektromagneten, ein Relais, einen Schrittmotor, ein Piezoelement, einen elektromechanischen Aktor oder einen Servomotor. Derart ausgestattete Antriebe gestatten es insbesondere, die Sicherungseinrichtung nicht nur mechanisch zu betätigen, sondern auch elektrisch oder elektronisch anzusteuern.

Auf einfache Weise lässt sich Körpergewebe durchtrennen, wenn das Schneidelement ein mechanisches Schneidelement oder ein HF-Schneidelement ist. Zum einen kann ein mechanisches Schneidelement in Form einer Klinge zum Durchtrennen von Körpergewebe genutzt werden. Zum anderen ist das Durchtrennen von Körpergewebe auch mittels eine Stromes möglich. In diesem Fall ist eine Bewegung des Schneidelements relativ zu den Werkzeugelementen nicht zwingend erforderlich.

Vorteilhafterweise umfasst das Instrument eine Betätigungseinrichtung zum Bewegen der Werkzeugelemente relativ zueinander. Dies gestattet es einem Operateur auf einfache Weise, miteinander zu verbindendes Körpergewebe zu fassen und durch Beaufschlagen mit einem Strom zu verbinden. Ferner kann die Betätigungseinrichtung auch ein Betätigungselement zum Bewegen des Schneidelements umfassen, so dass ein Operateur das Schneidelement manuell beispielsweise aus einer gegenüber den Werkzeugelementen zurückgezogenen Stellung in eine gegenüber diesen vorgeschobene Stellung bewegen kann, so dass das Schneidelement mit dem zu durchtrennenden Körpergewebe in Kontakt treten kann. Allerdings kann diese Bewegung des Schneidelements nur erfolgen, wenn die Sicherungseinrichtung das Schneidelement nicht in der Gewebeverbindungsstellung sichert.

Die Handhabung des Instruments wird besonders einfach, wenn die Betätigungseinrichtung an einem proximalen Ende desselben angeordnet oder ausgebildet ist.

Grundsätzlich wäre es denkbar, das Schneidelement relativ zu mindestens einem der Werkzeugelemente oder aber auch zu beiden unbeweglich anzuordnen. Vorzugsweise ist das Schneidelement jedoch relativ zu einem der Werkzeugelemente bewegbar angeordnet. Dies gestattet es, das Schneidelement insbesondere von einer gegenüber dem mindestens einen Werkzeugelement zurückgezogenen Stellung in eine gegenüber diesem Werkzeugelement vorstehende Stellung zu überführen, um insbesondere das mechanische Durchtrennen von Körpergewebe mit dem Schneidelement, das beispielsweise in Form einer Schneidklinge mit einer Schneidekante ausgebildet sein kann, zu ermöglichen.

Günstig ist es, wenn das chirurgische System mindestens einen HF-Stromgenerator umfasst, welcher mit den HF-Elektroden und/oder dem Schneidelement wahlweise elektrisch leitend verbindbar ist. Mit dem HF-Stromgenerator kann somit Körpergewebe mit einem HF-Strom beaufschlagt werden, und zwar zum einen zum Verbinden des Körpergewebes und optional auch zum Durchtrennen desselben.

Vorteilhaft ist es, dass das chirurgische System mindestens eine Steuer-und/oder Regelungseinrichtung zum Steuern und/oder Regeln des HF-Stromgenerators und/oder des Instruments umfasst. Mit der Steuer- und/oder Regelungseinrichtung ist es insbesondere möglich, den HF-Stromgenerator beziehungsweise das Instrument so zu steuern und/oder zu regeln, dass das Körpergewebe in optimaler und definierter Weise versiegelt beziehungsweise verschweißt werden kann. Insbesondere kann dabei berücksichtigt werden, dass zum Vermeiden eines Versagens der Versiegelung beziehungsweise der Verschweißung die auf das Körpergewebe einwirkenden Parameter erfasst und geregelt werden.

Ein besonders kompakter Aufbau des chirurgischen Systems lässt sich insbesondere dadurch erreichen, dass der HF-Stromgenerator die mindestens eine Steuer- und/oder Regelungseinrichtung umfasst. Zum Beispiel können die Steuer- und/oder Regelungseinrichtung sowie der HF-Stromgenerator in einem gemeinsamen Gehäuse angeordnet sein.

Erfindungsgemäß ist die mindestens eine Steuer- und/oder Regelungseinrichtung zum Steuern der Sicherungseinrichtung ausgebildet. Dies gestattet ein definiertes Zusammenwirken der Steuer- und/oder Regelungseinrichtung sowie der Sicherungseinrichtung. Insbesondere kann auf diese Weise erreicht werden, dass beispielsweise eine Entsicherung des Schneidelements, die eine Bewegung desselben zum Durchtrennen von Körpergewebe ermöglicht, erst dann möglich ist, wenn eine erfolgreiche, durch die Steuer- und/oder Regelungseinrichtung gesteuerte Verbindung des Körpergewebes mittels eines HF-Stroms erfolgt ist.

Günstig ist es, wenn die Steuer- und/oder Regelungseinrichtung den mit dem Instrument verbundenen HF-Stromgenerator in einem Gewebeverbindungszyklus derart steuert und regelt, dass Körpergewebe zwischen den Elektroden definiert verbindbar ist. Durch eine solche Ausgestaltung der Steuer- und/oder Regelungseinrichtung kann sichergestellt werden, dass es nicht zu einem Versagen der Gewebeverbindung kommt. Insbesondere können bei der Verbindung des Körpergewebes ein oder mehrere auf dieses einwirkende Parameter erfasst und geregelt werden.

Vorteilhaft ist es, wenn am Instrument Sensoren zur Bestimmung von Verfahrensparametern angeordnet sind, und wenn die Steuer- und/oder Regelungseinrichtung die mit den Sensoren erfassten Verfahrensparameter beim Durchlaufen des Gewebeverbindungszyklus verarbeitet. Beispielsweise können mit den Sensoren eine Temperatur sowie ein Widerstand des Gewebes während des Gewebeverbindungszyklus bestimmt und abhängig von deren Werten eine Bestromung des HF-Stromgenerators entsprechend gesteuert und/oder geregelt werden, um eine definierte Versiegelung beziehungsweise Verschweißung des Körpergewebes zu erreichen sowie eine Zerstörung desselben zu vermeiden.

Vorzugsweise werden als Verfahrensparameter die Temperatur und/oder der Gewebewiderstand erfasst. Insbesondere kann durch Messung der genannten Verfahrensparameter auch in der Regel sehr zuverlässig ausgewertet werden, ob das Körpergewebe bereits, und falls ja, in gewünschter Weise verbunden wurde.

Gemäß einer bevorzugten Ausführungsform der Erfindung kann vorgesehen sein, dass die Steuer- und/oder Regelungseinrichtung die Sicherungseinrichtung derart steuert, dass das Schneidelement erst nach Abschluss eines Gewebeverbindungszyklus freigegeben wird, um es von der Gewebeverbindungsstellung in die Gewebetrennstellung bringen, insbesondere bewegen, zu können. Eine solche Ausbildung der Steuer- und/oder Regelungseinrichtung ermöglicht es, sicherzustellen, dass erst nach einer erfolgreich durchgeführten Verbindung des Körpergewebes das Schneidelement überhaupt zum Einsatz kommen und gegebenenfalls eine Durchtrennung oder Abtrennung mit diesem vorgenommen werden kann.

Vorteilhaft ist es, wenn die Steuer- und/oder Regelungseinrichtung derart ausgebildet ist, dass eine Bestromungsstärke und/oder eine Bestromungsdauer für die HF-Elektroden einstellbar ist. Mit einer solchen Steuer- und/oder Regelungseinrichtung lassen sich beispielsweise gewünschte Verfahrensparameter vorab einstellen und insbesondere als Sollwerte vorgeben.

Günstig ist es, wenn die Steuer- und/oder Regelungseinrichtung eine Temperaturmesseinrichtung umfasst zum Messen einer HF-Elektrodentemperatur und/oder einer Gewebetemperatur. Die HF-Elektrodentemperatur beziehungsweise die Gewebetemperatur als Verfahrensparameter zu messen, ermöglicht es insbesondere, bereits während des Gewebeverbindungszyklus das Fortschreiten sowie die Qualität der Gewebeverbindung zu ermitteln und zu bewerten.

Des Weiteren kann es vorteilhaft sein, wenn die Steuer- und/oder Regelungseinrichtung eine Widerstandsmesseinrichtung umfasst zum Messen eines Gewebewiderstands. Den Gewebewiderstand als Verfahrensparameter zu messen hat ebenfalls den Vorteil, dass so das Fortschreiten sowie eine Qualität der Gewebeverbindung auf einfache Weise ermittelt und bewertet werden können.

Die eingangs gestellte Aufgabe wird ferner bei einem nicht beanspruchtem Steuerungsverfahren der eingangs beschriebenen Art dadurch gelöst, dass das Schneidelement in der Gewebeverbindungsstellung so lange gesichert wird, bis ein Gewebeverbindungszyklus durchgeführt wurde, bei welchem Körpergewebe zwischen den Elektroden definiert verbunden wurde.

Das vorgeschlagene Steuerungsverfahren, welches insbesondere zum Steuern eines der oben beschriebenen chirurgischen Systeme verwendet werden kann, ermöglicht es auf einfache Weise, sicherzustellen, dass das Schneidelement erst dann aktiviert, insbesondere bewegt, und zum Durchtrennen von Körpergewebe zum Einsatz kommen kann, wenn ein ordnungsgemäßer, vorzugsweise vom chirurgischen System überwachter Gewebeverbindungszyklus durchgeführt wurde.

Die nachfolgende Beschreibung bevorzugter Ausführungsformen der Erfindung dient im Zusammenhang mit den Zeichnungen der näheren Erläuterung. Es zeigen:
Figur 1: eine schematische Darstellung eines erfindungsgemäßen chirurgischen Systems in einer Gewebeverbindungsstellung;

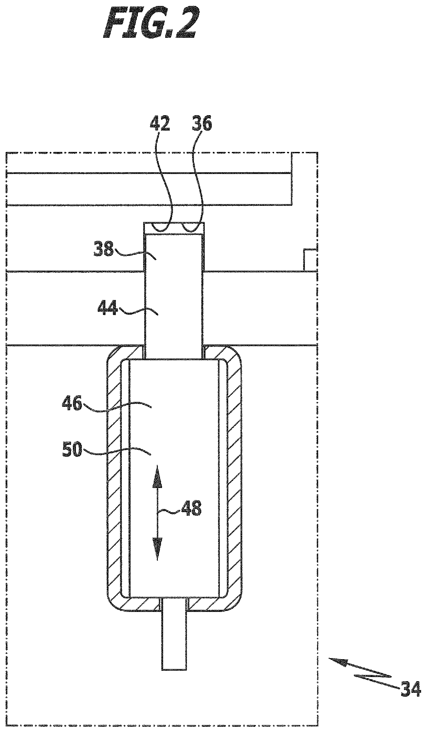

Figur 2: eine vergrößerte Ausschnittsansicht des Bereichs A in Figur 1 ; und
Figur 3: eine schematische Darstellung des in Figur 1 in einer Gewebeverbindungsstellung darstellten chirurgischen Instruments in einer Gewebetrennstellung.

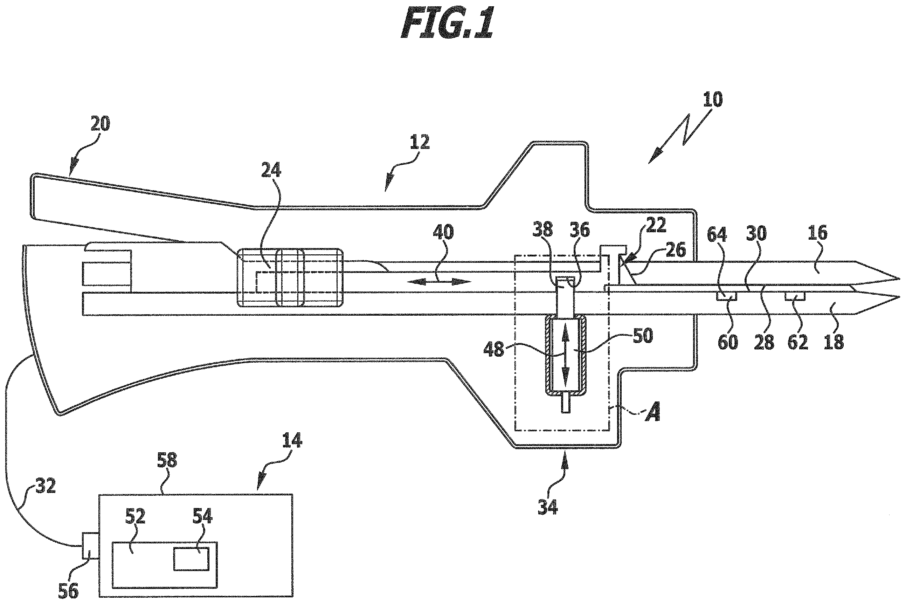

In Figur 1 ist schematisch ein erfindungsgemäßes chirurgisches System dargestellt und mit dem Bezugszeichen 10 bezeichnet. Es umfasst ein chirurgisches Instrument 12 und einen HF-Stromgenerator 14.

Das Instrument 12 umfasst zwei relativ zueinander bewegbare, vorzugsweise verschwenkbare Werkzeugelemente 16 und 18, die mittels einer ein proximales Ende des Instruments 12 bildenden Betätigungseinrichtung 20 bewegbar sind. Des Weiteren umfasst das Instrument 12 ein Schneidelement 22, welches in Form eines Schiebeskalpells ausgebildet ist. Es ist in nicht näher dargestellter Weise am Instrument 12 mittels einer Schneidelementhalterung verschiebbar gelagert. Zum Bewegen des Schneidelements 22 ist ein Schiebeknopf 24 vorgesehen, welcher von einer Außenseite des Instruments 12 vorstehend ausgebildet ist. Er kann beispielsweise mit einem Daumen eines Operateurs in distaler Richtung verschoben werden, wobei dann gleichzeitig das Schneidelement 22 mit einer in distaler Richtung weisenden Schneidkante 26 bewegt werden kann, und zwar relativ zu den Werkzeugelementen 16 und 18 derart, dass zumindest die Schneidkante 26 über diese vorsteht und so Körpergewebe durchtrennbar ist.

Die Werkzeugelemente 16 und 18 umfassen jeweils eine HF-Elektrode 28 beziehungsweise 30, die in einer Annährungsstellung der Werkzeugelemente 16 und 18 einen minimalen Abstand definieren, einander gegenüberliegen und aufeinander zu weisen. Sie können mit einem HF-Strom beaufschlagt werden, welcher vom HF-Stromgenerator 14 über eine Verbindungsleitung 32 zum Instrument 12 zugeleitet wird.

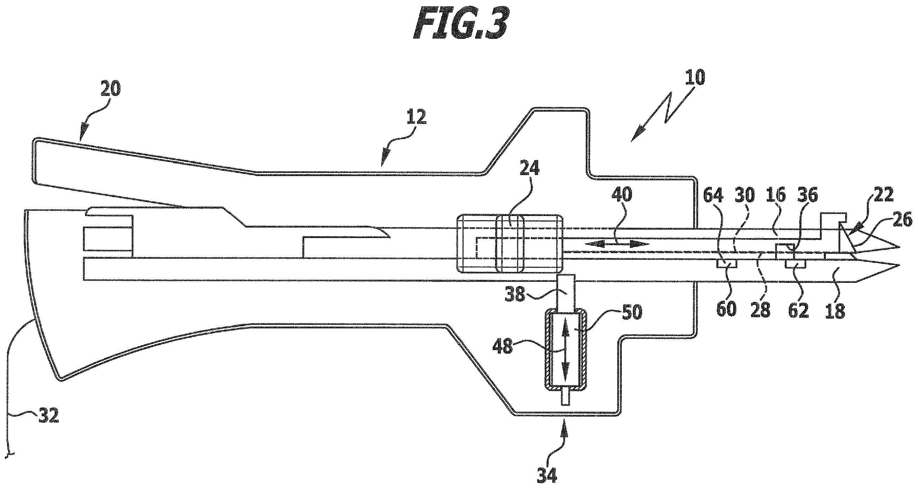

Des Weiteren umfasst das Instrument 12 eine Sicherungseinrichtung 34 zum Sichern des Schneidelements 22 in einer Gewebeverbindungsstellung, in welcher Körpergewebe mit den Werkzeugelementen 16, 18 verbindbar ist und in welcher das Schneidelemente 22 vorzugsweise nicht mit Körpergewebe in Kontakt treten kann. Die Sicherungseinrichtung 34 umfasst erste und zweite Sicherungselemente 36 und 38. Das erste Sicherungselement 36 ist vorzugsweise dem Schneidelement 22 zugeordnet oder ist von diesem umfasst beziehungsweise wird von diesem getragen. Das Instrument 12 umfasst ferner auch das zweite Sicherungselement 38 oder trägt dieses. Die Sicherungselemente 36, 38 sind relativ zueinander bewegbar ausgebildet und von einer Sicherungsstellung, wie schematisch in Figur 1 dargestellt, in welcher sie miteinander kraft- und/oder formschlüssig in Eingriff stehen, in eine Freigabestellung bringbar, wie sie schematisch in Figur 3 dargestellt ist, in welcher sie außer Eingriff stehen. Das erste Sicherungselement 36 ist in Form einer quer zu einer Verschieberichtung 40 des Schneidelements 22 weisend geöffnete Aussparung oder Ausnehmung 42 ausgebildet. Das zweite Sicherungselement 38 ist in Form eines bolzenförmigen Endes 44 ausgebildet, welcher in der Sicherungsstellung in die Ausnehmung 42 einführbar ist und dadurch eine Bewegung des Schneidelements 22 in der Verschieberichtung 40 blockiert.

Um die Sicherungselemente 36, 38 auf einfache Weise relativ zu einander bewegen zu können, ist das zweite Sicherungselement 38 mit einem Antrieb 46 gekoppelt. Dieser ermöglicht eine Bewegung des zweiten Sicherungselements 38 in einer Richtung 48, die quer, vorzugsweise senkrecht, zur Verschieberichtung 40 verläuft. Das zweite Sicherungselement 38 kann so auf einfache Weise in die Ausnehmung 42 hinein und auch wieder aus dieser heraus bewegt werden.

Der Antrieb 46 ist in Form eines magnetischen Antriebs 50 ausgebildet und umfasst einen Hubmagneten, welcher das bolzenförmige zweite Sicherungselement 38 in der Richtung 48 verschieben kann. In einer Grundstellung des Antriebs 46 nimmt das zweite Sicherungselement 38 die Sicherungsstellung ein. Ist also der Hubmagnet unbestromt, stehen die Sicherungselemente 36, 38 in Eingriff. Zum Überführen der Sicherungseinrichtung 34 von der Gewebeverbindungsstellung in die Freigabestellung muss der Antrieb 46 aktiviert werden, das heißt der Hubmagnet muss bestromt werden. Die Sicherungseinrichtung 34 ist somit in Form einer elektromechanischen Sperre ausgebildet. Als weitere Antriebsarten können alternativ auch elektromotorische, pneumatische, hydraulische oder auch piezoelektrische Antriebe statt des Magnetantriebs 50 vorgesehen sein.

Alternativ zu dem oben beschriebenen mechanischen Schneidelement 22 kann auch ein HF-Schneidelement vorgesehen. Dies bedeutet, dass zum Durchtrennen von Körpergewebe das Schneidelement 22 mit einem vom HF-Stromgenerator bereitgestellten HF-Strom beaufschlagt werden kann. In einem solchen Fall ist es nicht zwingend erforderlich, dass das Schneidelement 22 relativ zu den Werkzeugelementen 16, 18 bewegbar am Instrument 12 angeordnet ist. Der Schiebeknopf 24 kann dann insbesondere auch die Funktion eines elektrischen Schalters oder Tasters ausüben und muss nicht zwingend mechanisch mit dem Schneidelement 22 gekoppelt sein.

Zum Steuern und/oder Regeln des HF-Stromgenerators 14 dient eine Steuer-und/oder Regelungseinrichtung 52. Ebenfalls mit ihr steuerbar ist auch der Einsatz des Instruments 12. Die Steuer- und/oder Regelungseinrichtung 52 umfasst ferner vorzugsweise eine Schalteinrichtung 54 zum Schalten mindestens eines HF-Ausgangs 56 des Stromgenerators 14. Die Steuer- und/oder Regelungseinrichtung 52 kann insbesondere in einem Gehäuse 58 des HF-Stromgenerators 14 angeordnet sein.

Die Steuer- und/oder Regelungseinrichtung 52 ist derart ausgebildet, dass sie zum Steuern der Sicherungseinrichtung 34 eingesetzt werden kann. Dies wird dadurch erreicht, dass die Steuer- und/oder Regelungseinrichtung 52 die Sicherungseinrichtung 34 derart ansteuert, dass das Schneidelement 22 erst nach Abschluss eines ordnungsgemäßen Gewebeverbindungszyklus freigegeben wird. Während eines Gewebeverbindungszyklus kann Körpergewebe zwischen den Elektroden 28, 30 definiert durch Beaufschlagen mit HF-Strom verbunden werden. Dies steuert und/oder regelt die Steuer- und/oder Regelungseinrichtung 52, und zwar insbesondere unter Einbeziehung von am Instrument 12 angeordneten Sensoren 60 und 62 zur Bestimmung von Verfahrensparametern. Es kann sich dabei insbesondere um einen Sensor 60 in Form einer Temperaturmesseinrichtung 64 zum Bestimmen einer Temperatur der HF-Elektroden 28, 30 und/oder einer Gewebetemperatur handeln. Ferner kann der Sensor 62 in Form einer Widerstandsmesseinrichtung 66 zum Messen eines Gewebewiderstands ausgebildet sein. Die in der beschriebenen Weise erfassten Verfahrensparameter werden vorzugsweise mit der Steuer- und/oder Regelungseinrichtung 52 verarbeitet, um den Gewebeverbindungszyklus entsprechend zu steuern und/oder zu regeln. Durch Erfassen der Verfahrensparameter kann sichergestellt werden, dass das Gewebe definiert verbunden wird, um so ein späteres Versagen der Verbindung nach Möglichkeit ausschließen zu können. Ferner kann so auch eine Bestromung bei Bedarf derart begrenzt werden, dass eine Zerstörung des zu verbindenden Gewebes vermieden werden kann.

Um ferner eine Fehlbedienung des Systems 10 zu vermeiden, wird durch die Steuerung der Sicherungseinrichtung 34 durch die Steuer- und/oder Regelungseinrichtung 52 sichergestellt, dass das Schneidelement 22 erst nach Abschluss eines ordnungsgemäß durchlaufenen Gewebeverbindungszyklus freigegeben wird, um es aktivieren, beispielsweise von der Gewebeverbindungsstellung in die Gewebetrennstellung bewegen oder mit einem HF-Strom zum Schneiden beaufschlagen zu können.

Durch die Steuer- und/oder Regelungseinrichtung 52 können einzelne Funktionen des Systems 10 also insbesondere mittels Sicherheitsabfragen überwacht werden. Ein Gewebeverbindungszyklus kann dabei derart gesteuert werden, dass das Öffnen und Schließen, also das schlichte Bewegen der Werkzeugelemente 16 und 18 relativ zueinander, stets ermöglicht wird. Ferner kann es auch stets zugelassen werden, die Werkzeugelemente 16 und 18 in die Gewebeverbindungsstellung zu überführen, das Gewebe zu verbinden und anschließend die Werkzeugelemente 16 und 18 wieder zu öffnen, um das zwischen Ihnen gefasste Gewebe freizugeben. Das Schließen der Werkzeugelemente 16,18, das sich daran anschließende, durch den HF-Stromgenerator 14 gesteuerte und verifizierte Verbinden des Körpergewebes sowie das danach folgende Schneiden des Körpergewebes nach Freigabe des Schneidelements 22 durch die Sicherungseinrichtung 34 soll ebenfalls möglich sein. Nicht dagegen zulässig ist das Schließen der Werkzeugelemente 16, 18 und eine sich daran anschließende direkte Aktivierung des Schneidelements 22 ohne eine erfolgreiche, von der Steuer- und/oder Regelungseinrichtung 52 gesteuerte HF-Koagulation. Ebenfalls nicht möglich soll das Aktivieren des Schneidelements 22 sein, wenn nach Schließen der Werkzeugelemente 16, 18 eine Verbindung des Körpergewebes stattgefunden hat, die jedoch nicht durch die Steuer- und/oder Regelungseinrichtung 52 gesteuert wurde. Da eine Koagulation mit einem nicht zum System 10 gehörenden Gerät nicht mit dem System verifizierbar ist, bleibt die Sicherungseinrichtung 34 derart deaktiviert, dass das Schneidelement 22 nicht freigegeben wird, also weder bewegt noch betätigt werden kann.

Die oben beschriebenen bevorzugten Ausführungsformen eines chirurgischen Systems 10 sowie eines chirurgischen Steuerungsverfahrens ermöglichen einen geregelten Operations-Prozess, mit dem ein Operateur in die Lage versetzt wird, Gewebeanastomosen sicher herstellen zu können. Es kann insbesondere mit den beschriebenen Systemen und Verfahren erreicht werden, dass das mit dem Instrument 12 gefasste Gewebe nicht durchtrennt werden kann, bevor die Steuer- und/oder Regelungseinrichtung 52 den Gewebeverbindungsprozess als für erfolgreich durchgeführt verifiziert hat.

Um verifizieren zu können, ob sich das Instrument 10 in der Gewebeverbindungsstellung oder der Schneidstellung befindet, kann optional beispielsweise eine nicht näher dargestellte induktive Überwachung des Hubmagneten integriert werden. Alternativ ist auch der Einsatz eines magnetischen Antriebs 50 in Form eines sogenannten bistabiler Hubmagnets möglich, welcher jeweils eine der beiden Stellungen, also entweder die Gewebeverbindungsstellung oder die Schneidstellung, stabil hält und durch in Folge einer Bestromung und durch Umpolen dann die jeweils andere Stellung einnimmt.

Ferner kann darüber hinaus noch eine Sicherung vorgesehen sein, welche beispielsweise über einen geeigneten Sensor, zum Beispiel einen Taster, eine Stellung der Betätigungseinheit 20 überwacht, um zu vermeiden, dass der Versiegelungsprozess bei geöffneter Betätigungseinheit 20 durchgeführt werden kann. Insbesondere kann die Betätigungseinheit 20 derart ausgestaltet werden, dass sie mit einem Betätigungsglied für die HF-Strom-Betätigung, beispielsweise einem HF-Taster, in einer logischen UND-Abfrage gekoppelt wird. Auf diese Weise kann sichergestellt werden, dass eine Bestromung der HF-Elektroden 28 nur bei geschlossener Betätigungseinheit und gleichzeitig aktiv gedrücktem HF-Taster möglich ist.

## Patentansprüche

1. Chirurgisches System (10) zum Verbinden und/oder Trennen von Körpergewebe mit einem chirurgischen Instrument (12), das zwei jeweils mit zumindest einer HF-Elektrode bestückten Instrumentenbranchen (16, 18) hat, die an einer Branchenhalterung, vorzugsweise einem Instrumentengriff für ein Öffnen und Schließen der Instrumentenbranchen (16, 18) gelagert sind und einem Schneidelement (22), das ebenfalls an der Branchenhalterung, vorzugsweise dem Instrumentengriff separat zu den Instrumentenbranchen (16, 18) gelagert ist, wobei das chirurgische Instrument (12) mit einer Sicherungseinrichtung (34) in Form eines Schneidelement-Blockiermechanismus ausgerüstet ist, der mindestens ein Sicherungselement (38) umfasst, welches in nicht aktiviertem oder unbetätigtem Zustand eine Schneidbewegung des Schneidelements (22) mechanisch vollständig verriegelt und aktiv in einen Entriegelungszustand überführbar ist, um eine Schneidbewegung zu erlauben, und wobei ein aktives Entriegeln der Sicherungseinrichtung (34) in mittels einer Steuer- und/oder Regelungseinrichtung (52) gesteuerter/geregelter Weise nur dann ermöglicht ist, wenn ein vordefinierter Gewebeverbindungszyklus abgeschlossen ist, und wenn die HF-Elektroden an den Instrumentenbranchen (16, 18) nicht bestromt sind, wobei die Sicherungseinrichtung (34) an einer solchen Position bezüglich des Schneidelements (22) angeordnet ist, dass dessen mindestens eine Sicherungselement (38) zumindest teilweise kraft- und/oder formschlüssig mit dem Schneidelement (22) selbst in Eingriff steht.

2. Chirurgisches System (10) nach dem vorstehenden Anspruch, **dadurch gekennzeichnet, dass** das mindestens eine Sicherungselement (38) der Sicherungseinrichtung (34) aus einem Riegel oder Bolzen (38) besteht, der in einem Winkel, vorzugsweise senkrecht zur Bewegungsrichtung des Schneidelements (22) beweglich ist und mit einer Hinterschneidung vorzugsweise in Form einer Ausnehmung (42) als ein weiteres Sicherungselement der Sicherungseinrichtung (34) für ein Verriegeln der Schneidelement-Bewegung in Eingriff bringbar ist, wobei die Hinterschneidung (42) vom Schneidelement (22) ausgebildet ist.

3. Chirurgisches System (10) nach Anspruch 2, **dadurch gekennzeichnet, dass** der Riegel oder Bolzen (38) mit einem Antrieb (46) wirkverbunden ist, der den Riegel oder Bolzen (38) in seine Verriegelungs- und Entriegelungsposition bewegt.

4. Chirurgisches System (10) nach Anspruch 3, **dadurch gekennzeichnet, dass** der Antrieb (46) der elektromagnetischen, pneumatischen, hydraulischen, magnetischen, elektromotorischen oder piezoelektrischen Bauart ist.

5. Chirurgisches System (10) nach einem der vorstehenden Ansprüche, **dadurch gekennzeichnet, dass** das Schneidelement (22) ein mechanisch wirkendes Skalpell oder ein HF-Messer ist.

6. Chirurgisches System (10) nach einem der vorstehenden Ansprüche, **dadurch gekennzeichnet, dass** die Steuer- und/oder Regelungseinrichtung (52) zur Steuerung/Regelung der HF-Strombeaufschlagung der HF-Elektroden angepasst ist.

7. Chirurgisches System (10) nach Anspruch 6 in Verbindung mit Anspruch 3 oder 4, **dadurch gekennzeichnet, dass** die Steuer- und/oder Regelungseinrichtung (52) den Antrieb (46) so steuert, dass ein Entriegeln der Sicherungseinrichtung (34) bei geschlossenen Branchen (16, 18) und bestromten HF-Elektroden (28, 30) blockiert ist.

## Claims

1. A surgical system (10) for bonding and/or transecting body tissue by a surgical instrument (12) having two instrument branches (16, 18) each equipped with at least one HF electrode which are supported on a branch holder, preferably an instrument handle for opening and closing the instrument branches (16, 18) and a cutting element (22) also supported on the branch holder, preferably the instrument handle, separately from the instrument branches (16, 18), wherein the surgical instrument (12) is equipped with a retaining means (34) in the form of a blocking mechanism of the cutting element which comprises at least one retaining element (38) which in the non-activated or non-operated condition mechanically completely locks a cutting movement of the cutting element (22) and can be actively transferred into an unlocking condition to permit a cutting motion, and wherein active unlocking of the retaining means (34) in a way controlled by a process control means (52) is only possible when a predefined tissue bonding cycle is finished, and when no current is fed to the HF electrodes at the instrument branches (16, 18), wherein the retaining means (34) is arranged with regard to the cutting element (22) at such a position that its at least one retaining element (38) is engaged at least partly with the cutting element (22) in a force-fit and/or form-fit manner.

2. The surgical system (10) according to the preceding claim, **characterized in that** the at least one retaining element (38) of the retaining means (34) consists of a locking bar or stud (38) which is movable at an angle, preferably perpendicularly to the direction of motion of the cutting element (22) and can be engaged in an undercut preferably in the form of a recess (42) as further retaining element of the retaining means (34) for locking the motion of the cutting element, wherein the undercut (42) is formed by the cutting element (22).

3. The surgical system (10) according to claim 2, **characterized in that** the locking bar or stud (38) is operatively connected to a drive (46) moving the locking bar or stud (38) into its locked and unlocked positions.

4. The surgical system (10) according to claim 3, **characterized in that** the drive (46) has an electromagnetic, pneumatic, hydraulic, magnetic, electromotive or piezoelectric design.

5. The surgical system (10) according to any one of the preceding claims, **characterized in that** the cutting element (22) is a mechanically acting scalpel or a HF knife.

6. The surgical system (10) according to any one of the preceding claims, **characterized in that** the process control means (52) is configured to control an HF current feed to the HF electrodes.

7. The surgical system (10) according to claim 6 in conjunction with claim 3 or 4, **characterized in that** the process control means (52) controls the drive (46) so that unlocking of the retaining means (34) is blocked when the branches (16, 18) are closed and current is fed to the HF electrodes (28, 30).

## Revendications

1. Système chirurgical (10) destiné à relier et/ou séparer des tissus corporels avec un instrument chirurgical (12) qui a deux branches d'instrument (16, 18) dotées respectivement d'au moins une électrode HF qui sont montées sur un support de branche, de préférence une poignée d'instrument pour une ouverture et une fermeture des branches d'instrument (16, 18), et un élément de coupe (22) qui est monté également sur le support de branche, de préférence la poignée d'instrument séparément par rapport aux branches d'instrument (16, 18), dans lequel l'instrument chirurgical (12) est équipé d'un système de sécurité (34) sous la forme d'un mécanisme de blocage de l'élément de coupe, qui comprend au moins un élément de sécurité (38) qui, à l'état non activé ou inactif, verrouille mécaniquement complètement un mouvement de coupe de l'élément de coupe (22) et peut être transféré activement dans un état de déverrouillage pour permettre un mouvement de coupe, et dans lequel un déverrouillage actif du système de sécurité (34) n'est alors possible de manière commandée/régulée au moyen d'un système de commande et/ou de régulation (52) que lorsqu'un cycle de liaison de tissu prédéfini est terminé, et lorsque les électrodes HF au niveau des branches d'instrument (16, 18) ne sont pas alimentées en courant, dans lequel le système de sécurité (34) est disposé au niveau d'une position relative à l'élément de coupe (22) de telle sorte que l'au moins un élément de sécurité (38) de celui-ci est lui-même en prise au moins partiellement par coopération de forces et/ou de formes avec l'élément de coupe (22).

2. Système chirurgical (10) selon la revendication précédente, **caractérisé en ce que** l'au moins un élément de sécurité (38) du système de sécurité (34) est constitué d'un verrou ou d'un boulon (38) qui est mobile dans un angle, de préférence de manière perpendiculaire à la direction de mouvement de l'élément de coupe (22), et peut être mis en prise avec une contre-dépouille, de préférence sous la forme d'un évidement (42), en tant qu'un autre élément de sécurité du système de sécurité (34) pour un verrouillage du mouvement de l'élément de coupe, dans lequel la contre-dépouille (42) est réalisée par l'élément de coupe (22).

3. Système chirurgical (10) selon la revendication 2, **caractérisé en ce que** le verrou ou le boulon (38) est relié activement avec un entraînement (46) qui déplace le verrou ou le boulon (38) dans sa position de verrouillage et de déverrouillage.

4. Système chirurgical (10) selon la revendication 3, **caractérisé en ce que** l'entraînement (46) est du type électromagnétique, pneumatique, hydraulique, magnétique, électromoteur ou piézoélectrique.

5. Système chirurgical (10) selon l'une quelconque des revendications précédentes, **caractérisé en ce que** l'élément de coupe (22) est un scalpel à action mécanique ou un couteau HF.

6. Système chirurgical (10) selon l'une quelconque des revendications précédentes, **caractérisé en ce que** le système de commande et/ou de régulation (52) est adapté pour la commande/régulation de l'alimentation en courant HF des électrodes HF.

7. Système chirurgical (10) selon la revendication 6 en relation avec la revendication 3 ou 4, **caractérisé en ce que** le système de commande et/ou de régulation (52) commande l'entraînement (46) de telle sorte qu'un déverrouillage du système de sécurité (34) est bloqué lorsque les branches (16, 18) sont fermées et les électrodes HF (28, 30) alimentées en courant.
